(19) Europäisches Patentamt
European Patent Office
Office européen des brevets

(11) Veröffentlichungsnummer: **0 209 622**
**B1**

(12) # EUROPÄISCHE PATENTSCHRIFT

(45) Veröffentlichungstag der Patentschrift: **07.06.89**

(51) Int. Cl.$^4$: **C 07 C 25/18**, C 09 K 19/30

(21) Anmeldenummer: **85115607.5**

(22) Anmeldetag: **17.03.83**

(60) Veröffentlichungsnummer der früheren Anmeldung nach Art. 76 EPÜ: **0094487**

(54) **Hydroterphenyle.**

(30) Priorität: **30.03.82 DE 3211601**

(43) Veröffentlichungstag der Anmeldung: **28.01.87 Patentblatt 87/05**

(45) Bekanntmachung des Hinweises auf die Patenterteilung: **07.06.89 Patentblatt 89/23**

(84) Benannte Vertragsstaaten: **AT CH DE FR GB IT LI NL**

(56) Entgegenhaltungen:
**EP-A- 0 019 665**
**EP-A- 0 062 470**
**DE-A- 3 139 130**
**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Römer, Michael, Dr.**
**Im Grossen Garten 18**
**D-6054 Rodgau 2 (DE)**
Erfinder: **Krause, Joachim, Dr.**
**Samuel-Morse-Strasse 14**
**D-6110 Dieburg (DE)**
Erfinder: **Eidenschink, Rudolf, Dr.**
**Kornblumenstrasse 1**
**D-6115 Münster (DE)**
Erfinder: **Weber, Georg**
**Wilhelm-Leuschner-Strasse 38**
**D-6106 Erzhausen (DE)**

**EP 0 209 622 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft neue Hydroterphenyle der Formel I

$$R^1—Cy—Cy—Ph—R^2 \quad (I)$$

worin
$R^1$ Alkyl,
$R^2$ F oder Cl,
Cy 1,4-Cyclohexylen und
Ph 1,4-Phenylen
bedeuten,
wobei die Alkylgruppe 1-10 C-Atome enthält.

Diese Substanzen können wie ähnliche, z. B. aus der DE-OS 29 33 563 bekannte Verbindungen als Komponenten flüssigkristalliner Dielektrika verwendet werden, insbesondere für Displays, die auf dem Prinzip der verdrillten Zelle beruhen.

In der EP-OS 0 019 665 ist eine sehr breite allgemeine Formel angegeben, die die Verbindungen der Formel I umschließt. Jedoch ist an keiner Stelle dieser EP-OS ein Hinweis zu finden, durch den die Herstellung und Verwendung dieser speziellen Verbindungen nahegelegt wird.

Die in dieser EP-OS offenbarten Verbindungen führen zu höheren Viskositätswerten und kleineren Mesophasenbereichen beim Zusatz zu Flüssigkristallbasis-mischungen als die Verbindungen der Formel I.

Der Anmelder hat sich unter Bezugnahme auf die ältere, nicht vorveröffentlichte Anmeldung DE 31 39 130 in der Bundesrepublik Deutschland freiwillig eingeschränkt und gesonderte Patentansprüche für den Vertragsstaat Deutschland vorgelegt.

Der Erfindung lag die Aufgabe zugrunde, neue stabile flüssigkristalline Verbindungen aufzufinden, die als Komponenten flüssigkristalliner Dielektrika geeignet sind, insbesondere für nematische Phasen mit breitem nematischem Bereich und niedriger Viskosität. Diese Aufgabe wurde mit der Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit breitem nematischem Bereich und niedriger Viskosität herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die Viskosität eines solchen Dielektrikums zu senken. Die Verbindungen der Formel I, insbesondere diejenigen, worin $R^2$, Cl bedeutet, eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch sind sie sehr stabil.

Gegenstand der Erfindung sind somit die Verbindungen der Formel I sowie ein Verfahren zu ihrer Herstellung dadurch gekennzeichnet, daß man eine der Formel I entsprechende Verbindung, die jedoch an Stelle von Wasserstoffatomen (eine) reduzierbare Gruppe(n) und/oder zusätzliche C—C-Bindungen enthalten, mit einem Reduktionsmittel behandelt.

Weiterhin ist Gegenstand der Erfindung die Verwendung der Verbindungen der Formel I als Komponenten flüssigkristalliner Dielektrika für electrooptische Anzeige-elemente. Gegenstand der Erfindung sind weiterhin flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, Cy und Ph die angegebene Bedeutung, sofern nicht ausdrücklich etwas anderes vermerkt ist.

In den Verbindungen der Formel I sind diejenigen Stereoisomeren bevorzugt, worin die Substituenten an den beiden 1,4-Cyclohexylenresten jeweils in trans-Stellung zueinander stehen.

In den Verbindungen der Formel I können die Alkylreste geradkettig oder verzweigt sein.

Vorzugsweise sind sie geradkettig, haben 2, 3, 4, 5 oder 6 C-Atome und bedeuten demnach bevorzugt Ethyl, Propyl, Butyl, Pentyl, Hexyl, ferner Methyl, Heptyl, Octyl, Nonyl oder Decyl.

Verbindungen der Formeln I verzweigter Flügelgruppe $R^1$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel

nicht mehr als eine Kettenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Heptyl (= 1-Methylhexyl), 2-Octyl (= 1-Methylheptyl), 2-Ethyl-pentyl.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden bevorzugt hergestellt, indem man eine Verbindung der Formel II.

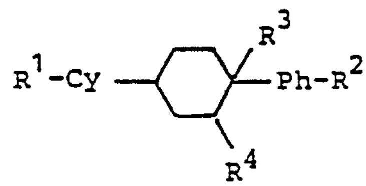

(II)

worin entweder $R^3$ OH und $R^4$ H
oder $R^3$ und $R^4$ zusammen eine C—C-Bindung
bedeuten,
reduziert.

Die Carbinole der Formel II ($R^3$ = OH, $R^4$ = H) sind beispielsweise erhältlich durch Hydrierung von Cyclohexyl-phenolen der Formel $R^1$—Cy—Ph—OH zu Cyclohexylcyclohexanolen der Formel $R^1$—Cy—Cy—OH, Oxydation zu den entsprechenden 4—$R^1$—Cy-Cyclohexanonen, Umsetzung mit einer Grignard-Verbindung der Formel BrMg—Ph—$R^2$ und Hydrolyse. Durch Wasserabspaltung, z. B. mit einer starken Säure wie Schwefelsäure oder p-Toluolsulfonsäure in einem inerten Lösungsmittel wie Benzol oder Toluol, können daraus die Cyclohexene der Formel II ($R^3$ und $R^4$ zusammen = C—C-Bindung) hergestellt werden.

Die Reduktion der Ausgangsstoffe der Formel II zu den Verbindungen der Formel I erfolgt zweckmäßig durch katalytische Hydrierung bei Temperaturen zwischen etwa 0° und etwa 200° sowie Drucken zwischen etwa 1 und etwa 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form (z. B. Pt-Mohr) eingesetzt werden können.

Nach der Reduktionsstufe kann es zweckmäßig sein, das erhaltene Stereoisomerengemisch in das stabile trans-Isomere (bezogen auf den durch die Hydrierung erhaltenen Cyclohexanring) umzuwandeln, z. B. durch Behandeln mit einer Base wie K-tert.-Butylat in einem inerten Lösungsmittel wie Dimethylformamid (DMF), N-Methylpyrrolidon oder Dimethylsulfoxid bei Temperaturen zwischen etwa 0 und etwa 150°.

Gewünschtenfalls kann man in einer erhaltenen Verbindung der Formel I den Rest $R^2$ in einen anderen Rest $R^2$ umwandeln.

So führt eine Chlorierung der Hydroterphenyle der Formel I mit $R^2$ = H zu Chlor- verbindungen der Formel I ($R^2$ = Cl), beispielsweise mit elementarem Chlor in einem inerten Lösungsmittel wie Diethylether, $CCl_4$ oder Essigsäure bei Temperaturen zwischen etwa —30 und 100°, wobei Katalysatoren wie Eisenspäne, Jod oder $AlCl_3$ zugegen sein können.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel III charakterisieren,

$$R^6—A—G—E—R^7 \quad (III)$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | |
|---|---|
| G —CH = CH— | —N(O) = N— |
| —CH = CY— | —CH = N(O)— |
| —C ≡ C— | $—CH_2—CH_2—$ |
| —CO—O— | $—CH_2—O—$ |
| —CO—S— | $—CH_2—S—$ |
| —CH = N— | —COO—Ph—COO— |

oder eine C—C-Einfachbindung, Y Halogen, vorzugsweise Chlor, oder —CN, und $R^6$ und $R^7$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^6$ und $R^7$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 60, vorzugsweise 2 bis 25 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vergl. z. B. I. Haller et al., Mol. Cryst. Liq. Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz. Vor- und nachstehend bedeuten Prozentangaben Gewichtsprozent; alle Temperaturen sind in Grad Celsius angegeben. « Übliche Aufarbeitung » bedeutet: man gibt, falls erforderlich, Wasser und/oder ein organisches Lösungsmittel wie Toluol, $CH_2Cl_2$ oder $CHCl_3$ hinzu, trennt ab, dampft die organische Phase ein und reinigt den Rückstand durch Chromatographie und/oder Kristallisation.

## Beispiel 1

Eine Lösung von 30,0 g 1-p-Fluorphenyl-4-(trans-4-propyl-cyclohexyl)-cyclohexen [F. 59 °C, erhältlich durch Hydrierung von trans-1-p-Hydroxyphenyl-4-propyl-cyclohexan an $PtO_2$ in Ethylacetat bei 50° zu trans-4-(trans-4-Propylcyclohexyl)-cyclohexanol, Oxydation mit NaOCl zu 4-(trans-4-Propylcyclohexyl)-cyclohexanon, Reaktion mit $C_6H_4FMgBr$ in THF und nachfolgende Hydrolyse zu 1-p-Fluorphenyl-4-(trans-4-propylcyclohexyl)-cyclohexanol und Dehydratisierung mit p-Toluolsulfonsäure in siedendem Toluol] in 350 ml THF wird an 3 g PdO bei 40° und 1 bar bis zum Stillstand hydriert. Man filtriert, dampft ein, löst das erhaltene cis-trans-Gemisch in 140 ml DMF, versetzt mit 14 g K-tert.-Butylat und erhitzt 24 Stunden unter $N_2$ auf 100°.

Nach dem Abkühlen gießt man in Wasser und filtriert das erhaltene trans-1-p-Fluorphenyl-4-(trans-4-propylcyclohexyl)-cyclohexan ab. F. 80 °C, K. 158 °C.

Analog erhält man durch Hydrierung der entsprechenden Cyclohexene:

trans-1-p-Fluorphenyl-4-(trans-4-ethylcyclohexyl)-cyclohexan
trans-1-p-Fluorphenyl-4-(trans-4-butylcyclohexyl)-cyclohexan, F. 78°, K. 152°
trans-1-p-Fluorphenyl-4-(trans-4-pentylcyclohexyl)-cyclohexan, F. 69°, K. 157°
trans-1-p-Fluorphenyl-4-(trans-4-hexylcyclohexyl)-cyclohexan, F. 68°, K. 145°
trans-1-p-Chlorphenyl-4-(trans-4-ethylcyclohexyl)-cyclohexan
trans-1-p-Chlorphenyl-4-(trans-4-propylcyclohexyl)-cyclohexan, F. 75°, K. 192°
trans-1-p-Chlorphenyl-4-(trans-4-butylcyclohexyl)-cyclohexan, F. 57°, K. 182°
trans-1-p-Chlorphenyl-4-(trans-4-pentylcyclohexyl)-cyclohexan, F. 62°, K. 183°
trans-1-p-Chlorphenyl-4-(trans-4-hexylcyclohexyl)-cyclohexan, F. 58°, K. 173°

Es folgen Beispiele für erfindungsgemäße Dielektrika mit einem Gehalt an mindestens einer Verbindung der Formel I:

Beispiel A

Ein Gemisch aus
24 % p-(trans-4-Propylcyclohexyl)-benzonitril
36 % p-(trans-4-Pentylcyclohexyl)-benzonitril
25 % p-(trans-4-Heptylcyclohexyl)-benzonitril
15 % trans-1-p-Fluorphenyl-4-(trans-4-propylcyclohexyl)-cyclohexan
zeigt F. — 15°, K. 62°.

Es kann z. B. verwendet werden für einen « Twisted Nematic Display » (TND) bei Ansteuerung im « Static Drive » (« Direktansteuerung ») mit Betriebsspannungen von 3-10 Volt.

Beispiel B

Ein Gemisch aus
12 % p-(trans-4-Propylcyclohexyl)-benzonitril
10 % p-(trans-4-Butylcyclohexyl)-benzonitril
19 % p-(trans-4-Pentylcyclohexyl)-benzonitril
10 % p-(trans-4-Heptylcyclohexyl)-benzonitril
12 % 4-Cyan-4′-(trans-4-pentylcyclohexyl)-biphenyl
12 % trans-1-p-Cyanphenyl-4-(trans-4-ethylcyclohexyl)-cyclohexan
15 % trans-1-p-Cyanphenyl-4-(trans-4-propylcyclohexyl)-cyclohexan
10 % trans-1-p-Fluorphenyl-4-(trans-4-propylcyclohexyl)-cyclohexan
zeigt F. — 13°, K. 97°.

Es ist z. B. als nematisches Lösungsmittel für dichroitische Farbstoffe geeignet (« Hostmischung »).

**Patentansprüche** (für die Vertragsstaaten : FR, GB, IT, NL, CH, LI)

1. Hydroterphenyle der Formel I

$$R^1—Cy—Cy—Ph—R^2 \qquad (I)$$

worin
$R^1$ Alkyl,
$R^2$ F oder Cl,
Cy 1,4-Cyclohexylen und
Ph 1,4-Phenylen
bedeuten,
wobei die Alkylgruppe 1-10 C-Atome enthält.

2. Verfahren zur Herstellung von Hydroterphenylen der Formel I nach Anspruch 1, dadurch gekennzeichnet, daß man eine der Formel I entsprechende Verbindung, die jedoch an Stelle von Wasserstoffatomen (eine) reduzierbare Gruppe(n) und/oder zusätzliche C—C-Bindungen enthalten, mit einem Reduktionsmittel behandelt.

3. Verwendung der Verbindungen der Formel I nach Anspruch 1 als Komponenten flüssigkristalliner Dielektrika für elektrooptische Anzeigeelemente.

4. Flüsigkristallines Dielektrikum für elektrooptische Anzeigeelemente mit mindestens zwei flüssigkristallinen Komponenten, dadurch gekennzeichnet, daß mindestens eine Komponente eine Verbindung der Formel nach Anspruch 1 ist.

5. Elektrooptisches Anzeigelement, dadurch gekennzeichnet, daß es ein Dielektrikum nach Anspruch 4 enthält.

**Patentansprüche** (für den Vertragsstaat DE)

1. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I

$$R^1—Cy—Cy—Ph—R^2 \qquad (I)$$

worin
$R^1$ Alkyl,
$R^2$ F oder Cl,
Cy 1,4-Cyclohexylen und
Ph 1,4-Phenylen
bedeuten,

wobei die Alkylgruppe 1-10 C-Atome enthält,
ist und es mindestens einen Bestandteil der Formel III enthält,

$$R^6—A—G—E—R^7 \qquad (III)$$

worin

A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G —CH = CH—
—CH = CY—
—C ≡ C—
—CO—O—
—CO—S—
—CH = N—
—N(O) = N—
—CH = N(O)—
$—CH_2—CH_2—$
$—CH_2—O—$
$—CH_2—S—$
—COO—Ph—COO—

Y Halogen oder —CN, und

$R^6$ und $R^7$ Alkyl, Alkoxy, Alkanoyloxy oder Alkoxycarbonyloxy mit bis zu 8 Kohlenstoffatomen, oder einer dieser Reste auch CN, NC, $NO_2$, $CF_3$, F, Cl oder Br bedeuten.

2. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I

$$R^1—Cy—Cy—Ph—R^2 \qquad (I)$$

worin

$R^1$ Alkyl,
$R^2$ F oder Cl,
Cy 1,4-Cyclohexylen und
Ph 1,4-Phenylen

bedeuten,
wobei die Alkylgruppe 1-10 C-Atome enthält,
ist und es mindestens einen anderen Bestandteil enthält, ausgewählt aus den Klassen der Azoxybenzole, Benzylidenaniline, Biphenyle, Terphenyle, Phenyl- oder Cyclohexylbenzoate, Cyclohexancarbonsäurephenyl- oder cyclohexyl-ester, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren.

3. Elektrooptisches Anzeigelement, dadurch gekennzeichnet, daß es ein Dielektrikum nach einem der Ansprüche 1 oder 2 enthält.

**Patentansprüche** (für den Vertragsstaat AT)

1. Verfahren zur Herstellung von Hydroterphenylen der Formel I

$$R^1—Cy—Cy—Ph—R^2 \qquad (I)$$

worin

$R^1$ Alkyl,
$R^2$ F oder Cl,
Cy 1,4-Cyclohexylen und
Ph 1,4-Phenylen

bedeuten,
wobei die Alkylgruppe 1-10 C-Atome enthält,
dadurch gekennzeichnet, daß man eine der Formel I entsprechende Verbindung, die jedoch an Stelle von Wasserstoffatomen (eine) reduzierbare Gruppe(n) und/oder zusätzliche C—C-Bindungen enthalten, mit einem Reduktionsmittel behandelt.

2. Verwendung der Verbindungen der Formel I nach Anspruch 1 als Komponenten flüssigkristalliner Dielektrika für elektrooptische Anzeigeelemente.

3. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

4. Elektrooptisches Anzeigelement, dadurch gekennzeichnet, daß es ein Dielektrikum nach nach Anspruch 3 enthält.

**Claims** (for the Contracting States : FR, GB, IT, NL, CH, LI)

1. Hydroterphenyls of the formula I

$$R^1—Cy—Cy—Ph—R^2 \quad (I)$$

wherein
$R^1$ is alkyl,
$R^2$ is F or Cl,
Cy is 1,4-cyclohexylene and
Ph is 1,4-phenylene,
and the alkyl group contains 1-10 C atoms.

2. Process for the preparation of hydroterphenyls of the formula I according to Claim 1, characterised in that a compound which corresponds to the formula I but contains (a) reduceable group(s) and/or additional C—C bonds instead of hydrogen atoms, is treated with a reducing agent.

3. Use of the compounds of the formula I according to Claim 1 as components of liquid crystal dielectrics for electrooptical display elements.

4. Liquid crystal dielectric for electrooptical display elements with at least two liquid crystal components, characterised in that at least one component is a compound of the formula I according to Claim 1.

5. Electrooptical display element, characterised in that it contains a dielectric according to Claim 4.

**Claims** (for the Contracting State : DE)

1. Liquid crystal dielectric for electrooptical display elements with at least two liquid crystal components, characterised in that at least one component is a compound of the formula I

$$R^1—Cy—Cy—Phe—R^2 \quad (I)$$

wherein
$R^1$ is alkyl,
$R^2$ is F or Cl,
Cy is 1,4-phenylene,
and the alkyl group contains 1-10 C atoms,
and
at least one component is a compound of the formula III,

$$R^6—A—G—E—R^7 \quad (III)$$

wherein
A and E are a carbocyclic or heterocyclic ring system from the group comprising 1,4-disubstituted benzene and cyclohexene rings, 4,4′-disubstituted biphenyl-, phenylcyclohexane- and cyclohexylcyclohexane systems, 2,5-disubstituted pyrimidine and 1,3-dioxane rings, 2,6-disubstituted naphthalene, di- and tetrahydronaphthalene, quinazoline and tetrahydroquinazoline,
G is —CH = CH—, —CH = CY, —C ≡ C—, —CO—O—, —CO—S—, —CH = N—, —N(O) = N—, —CH = N(O)—, $—CH_2CH_2—$, $—CH_2—O—$, $—CH_2—S—$, —COO—Ph—COO—,
Y is a halogen or —CN and
$R^6$ and $R^7$ are alkyl, alkoxy, alkanoyloxy or alkoxycarbonyloxy with up to 8 carbon atoms or one of these radicals can also be CN, NC, $NO_2$, $CF_3$, F, Cl or Br.

2. Liquid crystal dielectric for electrooptical display elements with at least two liquid crystal components, characterised in that at least one component if a compound of the formula I

$$R^1—Cy—Cy—Ph—R^2 \quad (I)$$

wherein
$R^1$ is alkyl
$R^2$ is F or Cl
Cy is 1,4-cyclohexylene and
Ph is 1,4-phenylene
and the alkyl group contains 1-10 C atoms,
and

at least one other component is chosen from the classes of azoxybenzenes, benzylideneanilines, biphenyles, terphenyles, phenyl or cyclohexyl benzoates, phenyl or cyclohexyl cyclohexane carboxylates, cyclohexylbiphenyles, cyclohexylcyclohexanes, cyclohexylnaphthalenes, 1,4-bis-cyclohexylbenzenes, phenyl- or cyclohexyldioxanes, optionally halogenated stilbenes, benzylphenylethers, tolanes, or substituted cinnamic acids.

3. Electrooptical display element, characterised in that it contains a dielectric according to one of the Claims 1 or 2.

**Claims** (for the Contracting State AT)

1. Process for the preparation of hydroterphenyls of the formula I

$$R^1\text{—Cy—Cy—Ph—}R^2 \qquad (I)$$

wherein

$R^1$ is alkyl,
$R^2$ is F or Cl,
Cy is 1,4-cyclohexylene and
Ph is 1,4-phenylene,

and the alkyl group contains 1-10 C atoms, characterised in that a compound which corresponds to the formula I but contains (a) reduceable group(s) and/or additional C—C bonds instead of hydrogen atoms, is treated with a reducing agent.

2. Use of the compounds of the formula I according to Claim 1 as components of liquid crystal dielectrics for electrooptical display elements.

3. Liquid crystal dielectric for electrooptical display elements with at least two liquid crystal components, characterised in that at least one component is a compound of the formula I according to Claim 1.

4. Electrooptical display element, characterised in that it contains a dielectric according to Claim 3.

**Revendications** (pour les Etats contractants : FR, GB, IT, NL, CH, LI)

1. Hydroterphényles de formule I

$$R^1\text{—Cy—Cy—Ph—}R^2 \qquad (I)$$

où

$R^1$ représente un alkyle,
$R^2$ représente F ou Cl,
Cy représente le 1,4-cyclohexylène et
Ph représente le 1,4-phénylène,

le groupe alkyle contenant 1 à 10 atomes de carbone.

2. Procédé de préparation des hydroterphényles de formule I selon la revendication 1, caractérisé en ce que l'on traite un composé correspondant à la formule I, ledit composé contenant toutefois à la place d'atomes d'hydrogène un ou des groupe(s) réductible(s) et/ou des liaisons C—C supplémentaires, avec un réducteur.

3. Utilisation des composés de formule I selon la revendication 1, comme composants de diélectriques à cristaux liquides pour éléments d'affichage électro-optiques.

4. Diélectrique à cristaux liquides pour éléments d'affichage électro-optiques avec au moins deux composants à cristaux liquides, caractérisé en ce qu'au moins un composant est un composé de formule I selon la revendication 1.

5. Elément d'affichage électro-optique, caractérisé en ce qu'il contient un diélectrique selon la revendication 4.

**Revendications** (pour l'Etat contractant DE)

1. Diélectrique à cristaux liquides pour éléments d'affichage électro-optiques avec au moins deux composants à cristaux liquides, caractérisé en ce qu'au moins un composant est un composé de formule I

$$R^1\text{—Cy—Cy—Ph—}R^2 \qquad (I)$$

où

$R^1$ représente un alkyle,
$R^2$ représente F ou Cl,
Cy représente le 1,4-cyclohexylène et

Ph représente le 1,4-phénylène,

le groupe alkyle contenant 1 à 10 atomes de C, et en ce qu'il contient au moins un constituant de formule III,

$$R^6—A—G—E—R^7 \qquad (III)$$

où

A et E représentent une combinaison carbo- ou hétérocyclique provenant du groupe formé par les noyaux benzène et cyclohexane disubstitués en 1,4, les combinaisons biphényle, phényle, cyclohexane et cyclohexylcyclohexane disubstitués en 4,4', les noyaux pyrimidine et 1,3-dioxanne disubstitués en 2,5, la naphtalène disubstituée en 2,6, la di- et tétrahydronaphtalène, la quinazoline et la tétrahydroquinazoline,

| | |
|---|---|
| G —CH = CH— | —N(O) = N— |
| —CH = CY— | —CH = N(O)— |
| —C ≡ C— | $—CH_2—CH_2—$ |
| —CO—O— | $—CH_2—O—$ |
| —CO—S— | $—CH_2—S—$ |
| —CH = N— | —COO—Ph—COO— |

Y un halogène ou —CN, et

$R^6$ et $R^7$ un alkyle, un alcoxy, un alcanoyloxy ou un alcoxycarbonyloxy avec jusqu'à 8 atomes de carbone ou l'un de ces restes également CN, NC, $NO_2$, $CF_3$, F, Cl ou Br.

2. Diélectrique à cristaux liquides pour éléments d'affichage électro-optiques avec au moins deux composants à cristaux liquides, caractérisé en ce qu'au moins un composant est un composé de formule I

$$R^1—Cy—Cy—Ph—R^2 \qquad (I)$$

où

$R^1$ représente un alkyle,

$R^2$ représente F ou Cl,

Cy représente le 1,4-cyclohexylène et

Ph représente le 1,4-phénylène,

le groupe alkyle contenant 1 à 10 atomes de C, et en ce qu'il contient au moins un autre constituant choisi dans les classes des azoxybenzènes, benzylidèneanilines, biphényles, terphényles, benzoates de phényle et de cyclohexyle, phényl- ou cyclohexyl-esters de l'acide cyclohexanecarboxylique, cyclohexylbiphényles, cyclohexylcyclohexanes, cyclohexylnaphtalènes, 1,4-bis-cyclohexylbenzènes, 4,4'-bis-cyclohexylbiphényles, phényl- ou cyclohexyl-pyrimidines, phényl- ou cyclohexyldioxannes, éventuellement des stilbènes halogénés, des benzylphényléthers, des tolanes et des acides cinnamiques substitués.

3. Elément d'affichage électro-optique, caractérisé en ce qu'il contient un diélectrique selon l'une des revendications 1 ou 2.

**Revendications** (pour l'Etat contractant AT)

1. Procédé de préparation d'hydroterphényles de formule I

$$R1—Cy—Cy—Ph—R^2$$

où

$R^1$ représente un alkyle,

$R^2$ représente F ou Cl,

Cy représente le 1,4-cyclohexylène et

Ph représente le 1,4-phénylène,

le groupe alkyle contenant 1 à 10 atomes de C, caractérisé en ce que l'on traite un composé correspondant à la formule I, ledit composé contenant toutefois à la place d'atomes d'hydrogène un ou des groupe(s) réductible(s) et/ou des liaisons C—C supplémentaires, avec un réducteur.

2. Utilisation de composés de formule I selon la revendication 1, comme composants de diélectriques à cristaux liquides pour éléments d'affichage électro-optiques.

3. Diélectrique à cristaux liquides pour éléments d'affichage électro-optiques avec au moins deux composants à cristaux liquides, caractérisé en ce qu'au moins un composant est un composé de formule I selon la revendication 1.

4. Elément d'affichage électro-optique, caractérisé en ce qu'il contient un diélectrique selon la revendication 3.